**Europäisches Patentamt**

(19) **European Patent Office**

**Office européen des brevets**

(11) Veröffentlichungsnummer: **0 112 974**
**B1**

(12) **EUROPÄISCHE PATENTSCHRIFT**

(45) Veröffentlichungstag der Patentschrift:
**25.11.87**

(51) Int. Cl.⁴: **A 61 K  6/06,** A 61 K  6/08

(21) Anmeldenummer: **83109717.5**

(22) Anmeldetag: **28.09.83**

(54) Grundiermasse.

(30) Priorität: **07.12.82  DE 3245172**
**07.09.83  DE 3332179**

(43) Veröffentlichungstag der Anmeldung:
**11.07.84 Patentblatt 84/28**

(45) Bekanntmachung des Hinweises auf die Patenterteilung:
**25.11.87 Patentblatt 87/48**

(84) Benannte Vertragsstaaten:
**AT CH DE FR GB LI SE**

(56) Entgegenhaltungen:
**EP-A-0 011 190**
**EP-A-0 053 442**
**US-A-3 329 648**

**CHEMICAL ABSTRACTS, Band 99, Nr. 18, 31.**
**Oktober 1983, Seite 80, Nr. 141572u, Columbus, Ohio,**
**US; T.S. YUSHCHENKO et al.: "UV curing of white**
**pigmented coatings"**

(73) Patentinhaber: **Kulzer & Co. GmbH, Philipp- Reis-**
**Strasse 8, D-6393 Wehrheim (TS.)1 (DE)**

(72) Erfinder: **Schaefer, Roland, Dr., Merianweg 5,**
**D-6382 Friedrichsdorf (DE)**

(74) Vertreter: **Heinen, Gerhard, Dr., Heraeusstrasse 12-**
**14, D-6450 Hanau/Main (DE)**

## Beschreibung

Die Erfindung betrifft eine Titandioxid als Pigment enthaltende Grundiermasse auf der Basis von polymerisierbarem Methacrylsäureester für dentale Zwecke.

Bei der Herstellung von künstlichen Zahnkronen und Brücken aus mit Kunststoff verblendetem Metall wird das Metallgerüst, um eine Beeinflussung der Farbe der Verblendung durch den metallischen Untergrund zu vermeiden und die Haftfestigkeit zwischen Kunststoffverblendung und Metall zu verbessern, mit einem undurchsichtigen - opaken - Material, bedeckt.

Ein für diesen Zweck geeignetes Material - bezeichnet als opake Grundiermasse - aus Titandioxid, Bariumsulfat, Aluminiumoxid, in flüssigem Polyglykoldimethacrylat gelöstem Polymer und Benzoylperoxid, das nach dem Auftragen in einer etwa 400 μm dicken Schicht auf das Metallgerüst durch Erhitzen gehärtet wird, ist aus der DE-B-1 516 453 bekannt.

Eine ein organisches Lösungsmittel enthaltende Kunststoffharzmischung zum Abdecken und Verblenden von Metallgerüsten und Stumpfmodellen wird in der DE-A-B-21 62 608 beschrieben. Die Kunstharzmischung besteht aus einem zum Beispiel in Nitromethan gelösten Copolymerisat und Titandioxid, Zinkoxid, Eisenoxid, Bariumsulfat und/oder Siliciumdioxid als Pigment.

Aus der EP-A-53 442 ist ein auch durch Bestrahlung mit UV-Licht oder sichtbarem Licht polymerisierbares flüssiges Dentalmaterial bekannt, das eine Mischung zweier Füllstoffe mit unterschiedlicher Teilchengröße enthält. Der die kleinere Teilchengröße aufweisende Füllstoff besteht zum Beispiel aus Siliciumdioxid, Titandioxid, Aluminiumoxid, röntgenopakem Füllstoff oder Mischungen daraus, der die größeren Teilchen aufweisende Füllstoff zum Beispiel aus Siliciumdioxid, Silicatglas, wie zum Beispiel Bariumaluminiumsilicat oder ein Glas, das ein Seltenerdmetalloxid oder ein anderes geeignetes Oxid, wie Lanthan-, Strontium-, Tantal-, Zirkonium-, Gadolinium-, Dysprosium-, Hafnium- oder Wolframoxid enthält. Andere Füllstoffe, wie Aluminiumoxid, Titandioxid, Calciumfluorid, können ebenfalls eingesetzt werden. Das Dentalmaterial eignet sich besonders als Zahnfüllungsmaterial, kann aber auch als dentale Glasur und dentaler Lack, als orthodontisches Klebemittel, als Zahnprothesenmaterial und als Kronen- und Brückenmaterial verwendet werden.

Die EP-A-11 190 betrifft Polymerisate aus Polyacrylaten oder Polymethacrylaten und einem gleichmäßig darin verteilten, anorganischen, feinteiligen Füllstoff, die als Füllmittel in pastenförmigen Dentalmassen geeignet sind. Die Füllstoffe sind in Wasser unlösliche, feinteilige anorganische Substanzen, zum Beispiel Metalloxide, -sulfate, -silikate und -phosphate sowie Gläser und keramische Massen und deren Mischungen, beispielsweise Magnesiumhydroxidcarbonat, Titandioxid, Bariumsulfat, Zirkondioxid oder Kieselsäure. Die Polymerisate werden durch Polymerisation der Monomeren, u.a. Ester der Acrylsäure und Methacrylsäure, auch sogenannte Urethanacrylate und -methacrylate, in Gegenwart der Füllstoffe und von zum Beispiel Benzoylperoxid oder Cyclohexylpercarbonat als Initiator hergestellt.

In der US-PS 3 329 648 wird ein Material aus Vinylpolymeren und gleichmäßig darin verteilten anorganischen photochromen Oxiden, die unter anderem aus dotiertem Titandioxid oder dotiertem Zirkoniumdioxid bestehen können, beschrieben.

Es ist Aufgabe der Erfindung, eine pigmenthaltige Grundiermasse auf Methacrylat-Basis zu finden, die frei von Lösungsmitteln ist und rasch ausgehärtet werden kann.

Die die Lösung der Aufgabe darstellende Grundiermasse ist erfindungsgemäß dadurch gekennzeichnet, daß sie aus aliphatischem Urethandimethacrylat, Butandioldimethacrylat, einem Photopolymerisationskatalysator-System aus Campherchinon und p-Dimethylaminoäthylbenzoat und 30 - 90 Gewichts-% einer Pigment-Mischung aus 50 - 67 Gewichts-% Zirkoniumdioxid und 33 - 50 Gewichts-% Titandioxid besteht.

Besonders bewährt hat sich diese Grundiermasse, wenn die Pigment-Mischung aus 67 Gewichts-% Zirkoniumdioxid und 33 Gewichts-% Titandioxid beziehungsweise aus 50 Gewichts-% Zirkoniumdioxid und 50 Gewichts-% Titandioxid zusammengesetzt ist.

Das in der Grundiermasse enthaltene Gemisch aus Campherchinon und p-Dimethylaminoäthylbenzoat gehört zur Gruppe der an sich bekannten, bei Bestrahlung sowohl mit UV-Licht als auch mit sichtbarem Licht wirksamen Photopolymerisationskatalysatoren aus Keton/Amin-Systemen, die zum Beispiel in der GB-A-1 408 265 beschrieben werden.

Die als Schicht auf das Metallgerüst aufgetragene Grundiermasse gemäß der Erfindung läßt sich durch Bestrahlen mit sichtbarem Licht oder mit UV-Licht in kurzer Zeit vollständig ausharten.

Die ausgehärtete Schicht besitzt selbst bei geringer Schichtdicke eine sehr gute Deckkraft, so daß die Farbe der Kunststoffverblendung nicht durch hindurchschimmerndes Metall beeinflußt wird. Sie haftet fest auf der Metalloberfläche und bewirkt einen festen Verbund von Metall und Kunststoffverblendung.

Die Grundiermasse gemäß der Erfindung, die gegebenenfalls durch Zusatz von Farbpigmenten eingefärbt werden kann, ist nicht nur zum Abdecken der Metallgerüste für mit Kunststoff zu verblendende Zahnkronen und Brücken geeignet, sondern auch zum Überziehen von zum Beispiel kosmetisch störende Verfärbungen aufweisenden natürlichen Zähnen und zum Ausbessern von beschädigtem Zahnersatz aus mit Porzellan verkleidetem Metall.

Die Herstellung einer Grundiermasse gemäß der Erfindung und ihre Polymerisation durch Bestrahlung mit sichtbarem Licht wird in den folgenden Beispielen beschrieben.

### Beispiel 1

12,2 Gewichts-% aliphatisches Urethandimethacrylat (Plex 6661 der Firma Röhm, Darmstadt),

11,5 Gewichts-% Butandioldimethacrylat, 0,8 Gewichts-% Campherchinon,

0,5 Gewichts-% p-Dimethylaminoäthylbenzoat,

50 Gewichts-% Zirkoniumdioxid,

25 Gewichts-% Titandioxid

werden innig miteinander vermischt. Die erhaltene Grundiermasse wird in ein Rohr (Innendurchmesser 6 mm, Höhe 3 mm) aus Delrin$^R$, einem Polyacetal-Kunststoff, gegeben und 2 Minuten lang mit dem Wolfram-Halogen-Lichtgerät Translux der Firma Kulzer & Co. GmbH bestrahlt. Nach Entfernen des unpolymerisiert gebliebenen Teils der Grundiermasse wird die Schichtdicke des durch Polymerisation ausgehärteten Teils gemessen. Sie beträgt 0,5 mm.

### Beispiel 2

24,5 Gewichts-% aliphatisches Urethandimethacrylat (Plex 6661 der Firma Röhm, Darmstadt),

24 Gewichts-% Butandioldimethacrylat,

1 Gewichts-% Campherchinon,

0,5 Gewichts-% p-Dimethylaminoäthylbenzoat,

25 Gewichts-% Zirkoniumdioxid,

25 Gewichts-% Titandioxid

werden innig miteinander vermischt. Die erhaltene Grundiermasse wird in ein Rohr (Innendurchmesser 6 mm, Höhe 3 mm) aus Delrin$^R$, einem Polyacetal-Kunststoff, gegeben und 2 Minuten lang mit dem Wolfram-Halogen-Lichtgerät Translux der Firma Kulzer & Co. GmbH bestrahlt. Nach Entfernen des unpolymerisiert gebliebenen Teils der Grundiermasse wird die Schichtdicke des durch Polymerisation ausgehärteten Teils gemessen. Sie beträgt 0,4 mm.

### Patentansprüche

1. Titandioxid als Pigment enthaltende Grundiermasse auf der Basis von polymerisierbarem Methacrylsäureester für dentale Zwecke, dadurch gekennzeichnet, daß sie aus aliphatischem Urethandimethacrylat, Butandioldimethacrylat, einem Photopolymerisationskatalysator-System aus Campherchinon und p-Dimethylaminoäthylbenzoat und 30 - 90 Gewichts-% einer Pigment-Mischung aus 50 - 67 Gewichts-% Zirkoniumdioxid und 33 - 50 Gewichts-% Titandioxid besteht.

2. Grundiermasse nach Anspruch 1, dadurch gekennzeichnet, daß die Pigment-Mischung aus 67 Gewichts-% Zirkoniumdioxid und 33 Gewichts-% Titandioxid besteht.

3. Grundiermasse nach Anspruch 1, dadurch gekennzeichnet, daß die Pigment-Mischung aus 50 Gewichts-% Zirkoniumdioxid und 50 Gewichts-% Titandioxid besteht.

4. Verwendung der Grundiermasse nach einem der Ansprüche 1 bis 3 zum Abdecken der Metallgerüste bei der Herstellung von künstlichen Zahnkronen und Brücken aus mit Kunststoff verblendetem Metall.

### Revendications

1. Matière d'apprêt à base d'ester méthacrylique polymérisable contenant du dioxyde de titane comme pigment pour applications dentaires, caractérisée en ce qu'elle consiste en uréthannediméthacrylate aliphatique, diméthacrylate de butanediol, un système catalyseur de photopolymérisation consistant en camphoquinone et p-diméthylaminobenzoate d'éthyle et 30-90 % en poids d'un mélange de pigments consistant en 50-67 % en poids de dioxyde de zirconium et 33-50 % en poids de dioxyde de titane.

2. Matière d'apprêt selon la revendication 1, caractérisée en ce que le mélange de pigments consiste en 67 % en poids de dioxyde de zirconium et 33 % en poids de dioxyde de titane.

3. Matière d'apprêt selon la revendication 1, caractérisée en ce que le mélange de pigments consiste en 50 % en poids de dioxyde de zirconium et 50 % en poids de dioxyde de titane.

4. Utilisation de la matière d'apprêt selon l'une des revendications 1 à 3 pour le recouvrement des carcasses métalliques dans la fabrication de couronnes dentaires artificielles et bridges en métal revêtu de matière plastique.

### Claims

1. Opaquing composition for dental purposes based on polymerisable methacrylic acid ester and containing titanium dioxide as a pigment, characterised in that it comprises aliphatic urethane dimethacrylate, butanediol dimethacrylate, a photopolymerisation catalyst system comprising camphor quinone and p-dimethyl amino ethylbenzoate and 30-90 weight % of a pigment mixture of 50-67 weight % of zirconium dioxide and 33-50 weight % of titanium dioxide.

2. Opaquing composition according to claim 1, characterised in that the pigment mixture comprises 67 weight % of zirconium dioxide and 33 weight % of titanium dioxide.

3. Opaquing composition according to claim 1, characterised in that the pigment mixture consists of 50 weight % of zirconium dioxide and 50 weight % of titanium dioxide.

4. Application of the opaquing composition according to one of claims 1 to 3, for covering the metal frameworks in the production of artificial tooth crowns and bridges from metal masked with plastics material.